# EUROPÄISCHE PATENTSCHRIFT

(11) **EP 1 699 387 B1**
(45) Veröffentlichungstag und Bekanntmachung des Hinweises auf die Patenterteilung: **23.02.2011**
(21) Anmeldenummer: 04802947.4
(22) Anmeldetag: 15.12.2004
(51) Int. Cl.: A61F 2/44

(54) **BAUTEILANORDNUNG UND BAUTEIL FÜR EINE PROTHESE**
PARTS ASSEMBLY AND PART FOR A PROSTHESIS
SYSTEME DE PIECES ET PIECE POUR UNE PROTHESE

(30) Priorität: 22.12.2003 DE 10361166; 09.06.2004 DE 102004027986
(43) Veröffentlichungstag der Anmeldung: 13.09.2006
(73) Patentinhaber: Meisel, Hans Jörg, 14163 Berlin (DE)
(72) Erfinder: Meisel, Hans Jörg, 14163 Berlin (DE)
(74) Vertreter: Bittner, Thomas L.
(86) Internationale Anmeldenummer: PCT/DE2004/002748
(87) Internationale Veröffentlichungsnummer: WO 2005/060878

(56) Entgegenhaltungen:
- EP-A- 0 747 025
- WO-A-03/059212
- DE-U1- 20 315 611
- FR-A- 2 239 980
- US-A- 6 113 638

## Beschreibung

Die Erfindung betrifft eine Halswirbel-Bandscheibenprothese und ein Bauteil für eine Halswirbelsäulen-Bandscheibenprothese.

Prothesen auf Basis eines oder mehrerer Bauteile werden genutzt, um Teile des Knochenskeletts, beispielsweise die Wirbelsäule oder ein Gelenk, in ihrer Funktionalität zu unterstützen oder sogar zu ersetzen.

Degenerative Schäden an der Halswirbelsäule, die mit einem Bandscheibenvorfall einhergehen oder durch knöcherne Einengung das Rückenmark komprimieren, in aller Regel, wenn eine Operationsindikation vorliegt, von ventral operativ angegangen. Bei diesem operativen Zugang muß, um eine Entlastung des Rückenmarkes und der Nervenwurzel zu erzielen, die Halsbandscheibe vollständig entfernt werden. Dies geht bislang bei dem erwähnten operativen Vorgehen naturgemäß mit einem Funktionsverlust des betroffenen Bewegungssegmentes einher. Um einen zusätzlichen Höhenverlust der Bandscheibe, der von sich aus schon zu einer Zunahme der degenerativen und neurologischen Veränderungen führen kann, zu vermeiden, war bislang die ventrale Fusionsoperation die Methode der Wahl.

Mit Hilfe von an die Bandscheibehöhe angepaßtem/n Knochenzement, Beckenkammknochen oder körbchenartigen Abstandshaltern - sogenannten Cages - wird hierbei zumindest die Bandscheibenhöhe rekonstruiert, wobei es weiteres Ziel dieses Vorgehens die knöcherne Fusion der Wirbelkörper des betroffenen Bewegungssegmentes ist. Diese hat den Nachteil, daß die Anschlußbewegungssegmente durch nach der Fusion einsetzender Hebelwirkung des geschaffenen Blockwirbels stärker als vorher in Mitleidenschaft gezogen werden, was einer zunehmenden Degeneration in diesen Anschlußsegmenten Vorschub leistet. Zukünftiges Ziel dieser Behandlungsmethodik ist bei geeigneter Indikation der segmentale Funktionserhalt durch eine Bandscheibenvollprothese.

Bisher vorgeschlagene Entwicklungen für Halswirbelsäule-Bandscheibenprothesen, die zu marktreifen Produkten geführt haben, sind in ihrem klinischen Einsatz nicht sehr weit verbreitet, weil aufwendige operative Vorbereitungen zur Implantation notwendig sind und diese mit irreversiblen Veränderungen an den Grund- und Deckplatten der betroffenen Wirbelkörper einhergehen. So müssen beispielsweise zum Einsatz der Prothese nach Bryan et al. Anteile der angrenzenden Wirbelkörper entfernt werden, um einerseits die Prothese zu fixieren, aber auch um das relativ hohe Implantat überhaupt zu interponieren. Grund für diese Höhenausdehnung dieses Implantats ist der sehr komplexe Aufbau, der eine Stoßdämpferfunktion erfüllt, und daß der Aufbau aus einer großen Anzahl von Einzelbauteilen besteht, die aus verschiedenen Materialien gefertigt sind. Der Implantationsaufwand ist mit über 30 Implantationsinstrumenten besonders zeitaufwendig. Die spontane Fusionsrate nach Implantation kann mit postoperativen Kortisongaben reduziert werden.

Das Dokument WO 03/059212 A1 offenbart eine Bandscheibenprothese aus zwei gelenkig miteinander verbundenen einstückigen Platten, welche aus Metall oder Polymer hergestellt sind.

Aus dem Dokument EP 0 747 025 A1 geht eine Bandscheibenprothese hervor, welche zwei gelenkig miteinander verbundene Platten aufweist, wobei die Platten beispielsweise aus einer Chromlegierung hergestellt sind.

Das Dokument DE 203 15 611 U1 beschreibt ein Zwischenwirbelimplantat mit einem ersten Verankerungsteil und einem zweiten Verankerungsteil, welche mittels eines Gleitkörpers und einer Lagerplatte gelenkig miteinander verbunden sind, wobei der Gleitkörper und die Lagerplatte beispielsweise aus Polyetherketonketon (PEEK) hergestellt sein können.

Aufgabe der Erfindung ist es, eine verbesserte Halswirbel-Bandscheibenprothese und ein verbessertes Bauteil für eine Halswirbel-Bandscheibenprothese anzugeben, die kostengünstig und mit möglichst geringem Fertigungsaufwand herstellbar sind.

Diese Aufgabe wird erfindungsgemäß durch eine Halswirbel-Bandscheibenprothese nach dem unabhängigen Anspruch 1 sowie ein Bauteil nach dem unabhängigen Anspruch 11 gelöst. Vorteilhafte Ausgestaltungen der Erfindung ergeben sich aus abhängigen Unteransprüchen. Die Grundbauteile und die Kopplungsbauteile sind aus einem Material, welches aus der folgenden Gruppe von Materialien ausgewählt ist: Polyetherketon (PEK), Polyacryletherketon (PAEK), Polyetheretherketon (PEEK), Polyetherketonketon (PEKK), Polyetherketonetherketonketon (PEKEKK) und Polyetherketonetherketon (PEKEK).

Ein Vorteil von PAEK liegt in dessen Werkstoffeigenschaften, d.h. des ähnlichen Elastizitätsmoduls gegenüber kortikalen Knochen. Zur weiteren Verbesserung der tribologischen und mechanischen Eigenschaften kann vorgesehen sein, PAEK mit einem Füllmaterial zu verwenden, beispielsweise Kohle- oder Glasfasern, und/oder die Polymermatrix zu modifizieren, zum Beispiel mittels Vernetzung oder einer Eisenimplantation.

Die einteilige Ausführung des Grundbauteils mit dem Kopplungsbauteil hat gegenüber bekannten Bauteilen für Halswirbelsäulen-Bandscheibenprothese, bei denen das Kopplungsbauteil an dem Grundbauteil montiert ist, den Vorteil, daß das Herstellungsverfahren vereinfacht wird, da Arbeitsschritte zur separaten Fertigung von Grundbauteil und Kopplungsbauteil sowie das anschließende Verbinden der beiden Teile entfallen. Das Bauteil für die Prothese kann als Gesamtbauteil in einem Herstellungsprozeß gefertigt werden. Dieses kann mit Hilfe eines Werkzeugs ausgeführt werden. Darüber hinaus werden Probleme einer stabilen und dauerhaften Lagerung des Kopplungsbauteils an dem Grundbauteil, wie sie bei der getrennten Fertigung und der nachfolgenden Montage der beiden Bauteile auftreten können, aufgrund der einteiligen Bauweise vermieden.

Da die Grundbauteile und die Kopplungsbauteile aus einem Material sind, ist es ermöglicht, bei der Fertigung ausschließlich Werkzeuge zu nutzen, mit denen das verwendete Material bearbeitet werden kann. Es besteht keine Notwendigkeit, unterschiedliche Werkzeuge für verschiedenen Materialien zu verwenden, was zur Kosteneinsparung führt.

Eine zweckmäßige Ausführungsform der Erfindung sieht vor, daß auf einer jeweiligen Außenseite der zwei Grundbauteile eine anatomisch angepaßte Kontaktfläche gebildet ist. Mit Hilfe der anatomisch angepaßten Kontaktfläche wird eine Implantation der Bauteilanordnung als Prothese im Skelett derart ermöglicht, daß die Prothese auf eine möglichst natürliche Weise in das Skelett integriert wird. Die anatomisch angepaßte Kontaktfläche unterstützt eine paßgenaue Integration der Prothese in das Skelett. Mit Hilfe der anatomisch angepaßten Kontaktfläche wird ein Verrutschen der Prothese behindert. Darüber hinaus wirkt das Anordnen des Knochens auf der anatomisch angepaßten Kontaktfläche bei der Implantieren der Prothese einer ungewünschten Verdrehung der Prothese relativ zu den Skelettknochen entgegen, die benachbart zur Prothese angeordnet sind, so daß eine stabile Lagerung der Prothese gefördert wird.

Zweckmäßig kann vorgesehen sein, daß an den zwei Grundbauteilen jeweils eine Verdrehsicherung gebildet ist, um ein Verdrehen der Grundbauteile relativ zu dem bei der Implantation benachbart zu den Grundbauteilen angeordneten Knochenteilen zu verhindern. Die Verdrehsicherung umfaßt vorzugsweise einen auf der jeweiligen Außenseite der Grundbauteile angeordneten Steg, in dem Durchbrüche vorgesehen sein können. In die Durchbrüche kann der Knochen hineinwachsen.

Um die Verlagerbarkeit der beiden Grundbauteile relativ zueinander zu ermöglichen, was bei der Nutzung der Bauteilanordnung in einer implantierten Prothese dann die Beweglichkeit der Knochenteile zueinander erlaubt, kann vorgesehen sein, daß die beiden Grundbauteile mit Hilfe einer Gleitverbindung miteinander gekoppelt sind. Die Gleitverbindung wird bevorzugt mit Hilfe von Gleitflächen an den Kopplungsbauteilen ausgeführt. Eine bevorzugte Ausgestaltung der Erfindung sieht vor, daß eine der Gleitflächen auf einem halbkugelförmigen Vorsprung an einem der Kopplungsbauteile gebildet ist. Nach dem Zusammenbau der Bauteilanordnung lagert die abgerundete Gleitfläche auf einer in ihrer Form hieran angepaßten Gegengleitfläche an einem anderen Kopplungsbauteil.

Um eine möglichst hohe Abriebfestigkeit von Gleitfläche und Gegengleitfläche zu erreichen, sind diese zweckmäßig mit einem Material auf Basis einer Chrom-Nickel-Legierung beschichtet.

Als Material für die Herstellung der Grundbauteile wird in einer Ausführungsform der Erfindung bevorzugt ein Poly-Ether-Ether-Keton genutzt. Dieses Material hat den Vorteil, daß hierdurch ein zu kortikalen Knochen ähnliches Elastizitätsmodul zur Verfügung gestellt wird.

Die Erfindung wird im folgenden anhand von Ausführungsbeispielen unter Bezugnahme auf eine Zeichnung näher erläutert. Hierbei zeigen:
- Fig. 1: eine perspektivische Darstellung eines Grundbauteils für eine Bauteilan- ordnung zur Verwendung als Prothese;
- Fig. 2: eine perspektivische Darstellung eines weiteren Grundbauteils für eine Bauteilanordnung zusammen mit dem Grundbauteil nach Fig. 1 zur Ver- wendung als Prothese;
- Fig. 3: eine perspektivische Darstellung einer Bauteilanordnung mit dem Grund- bauteil nach Fig. 1 und dem weiteren Grundbauteil nach Fig. 2 im gekop- pelten Zustand;
- Fig. 4A und 4B: eine Darstellung von zwei Knochenteilen, die mit Hilfe einer Bauteilan- ordnung nach Fig. 3 verbunden sind, in einem gekoppelten und einem ent- koppelten Zustand; und
- Fig. 5A und 5B: eine Darstellung von zwei weiteren Knochenteilen, die mit Hilfe einer Bauteilanordnung nach Fig. 3 verbunden sind, in einem gekoppelten und einem entkoppelten Zustand.

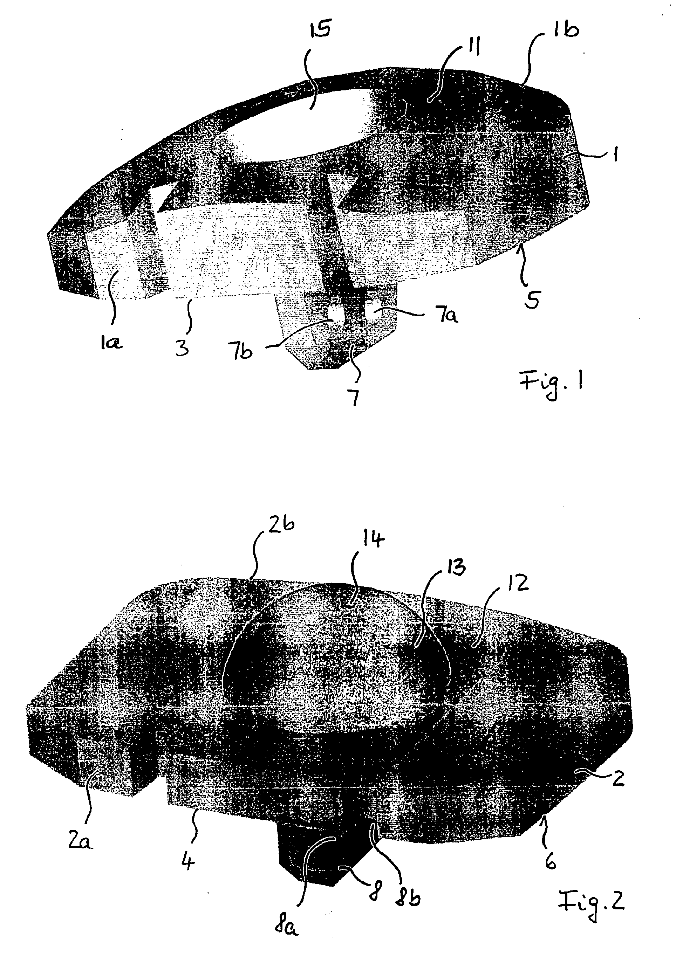

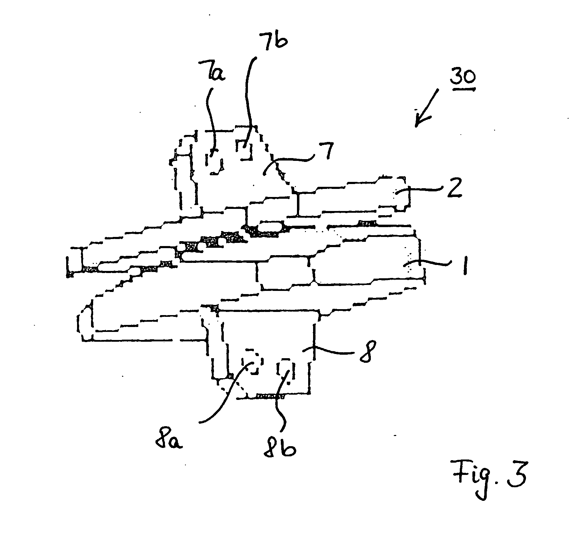

Die Fig. 1 und 2 zeigen eine perspektivische Darstellung eines Grundbauteils 1 und eines weiteren Grundbauteils 2 für eine Bauteilanordnung zur Verwendung als Prothese, insbesondere eine Halswirbelsäulen-Bandscheibenprothese. Fig. 3 zeigt eine perspektivische Darstellung einer Bauteilanordnung 30, in welcher das Grundbauteil 1 und das weitere Grundbauteil 2 gelenkig miteinander verbunden sind.

Gemäß Fig. 1 weist das Grundbauteil 1 einen dorsalen 1a und einen ventralen 1b Abschnitt auf. Auf einer Außenseite 3 ist eine anatomisch angepaßte Kontaktfläche 5 gebildet. Die anatomisch angepaßte Kontaktfläche 5 wird bei der Herstellung des Grundbauteils 1 an die Oberflächenkontur des Knochens angepaßt, in welchen die Prothese implantiert werden soll. Die Geometrie der Kontaktfläche 5 ermöglicht im Fall der Verwendung im Zusammenhang mit einer Bandscheibe eine möglichst gute kongruente Anpassung an die bei der Operation (Bandscheibenentfernung) sorgfältig vom Bandscheibenknorpel befreiten Deckplatten.

Gemäß Fig. 2 weist das weitere Grundbauteil 2 einen dorsalen 2a und einen ventralen 2b Abschnitt auf. Auf einer Außenseite 4 des Grundbauteils 2 ist eine anatomisch angepaßte Kontaktfläche 6 gebildet. Die anatomisch angepaßte Kontaktfläche 6 wird bei der Herstellung des Grundbauteils 2 an die Oberflächenkontur des Knochens angepaßt, in welchen die Prothese implantiert werden soll. Die Geometrie der Kontaktfläche 6 ermöglicht im Fall der Verwendung im Zusammenhang mit einer Bandscheibe eine möglichst gute kongruente Anpassung an die bei der Operation (Bandscheibenentfernung) sorgfältig vom Bandscheibenknorpel befreiten Deckplatten.

Für eine verbesserte Verhinderung einer Dislokation der Grundbauteile 1, 2 relativ zum Knochen kann eine Materialbeschichtung auf den anatomisch angepaßten Kontaktflächen 5, 6 vorgesehen sein, beispielsweise unter Verwendung von Hydroxylapatit.

Auf der Außenseite 3, 4 der Grundbauteile 1, 2 ist gemäß den Fig. 1 und 2 jeweils ein Steg 7, 8 angeordnet, welcher als Verdrehsicherung ausgebildet ist. Beim Implantieren der Bauteilanordnung greift der jeweilige Steg 7, 8 in eine Knochenvertiefung, so daß eine Verdrehung der Grundbauteile 1, 2 relativ zu dem jeweils benachbarten Knochen unterbunden ist. Es kann das Aufbringen einer Beschichtung auf die Stege 7, 8 vorgesehen sein, um eine verbesserte Verhinderung einer Dislokation der Prothese in situ zu erreichen. Die Stege 7, 8 verfügen über jeweilige Durchbrüche 7a, 7b bzw. 8a, 8b, durch die Knochen hindurch wachsen kann.

Die Grundbauteile 1, 2 verfügen gemäß den Fig. 1 und 2 über ein jeweiliges mit dem zugehörigen Grundbauteil 1, 2 einteilig ausgeführtes Kopplungsbauteil 11, 12. Mit Hilfe der Kopplungsbauteile 11, 12 wird eine Verbindung zwischen den beiden Grundbauteilen 1, 2 so hergestellt, daß die beiden Grundbauteile 1, 2 gelenkig miteinander verbunden sind. Zu diesem Zweck weist das Kopplungsbauteil 11 gemäß Fig. 1 ein Vorsprung 13 auf, welcher halbkugelförmig gestaltet ist. Auf dem Vorsprung 13 ist eine Gleitfläche 14 gebildet, die nach dem Zusammenbau der Bauteilanordnung (vgl. Fig. 3) auf einer Gegengleitfläche 15 an dem anderen Kopplungsbauteil 12 liegt, so daß zwischen den zwei Grundbauteilen 1, 2 eine gelenkige Gleitverbindung geschaffen ist. Mit Hilfe dieser Verbindung gewährleistet die Bauteilanordnung 30 bei der Verwendung als Prothese eine Beweglichkeit bzw. Gelenkigkeit des Skelettabschnitts, in welchen die Bauteilanordnung 30 als Prothese implantiert ist.

Die Grundbauteile 1, 2 und die Kopplungsbauteile 11, 12 können hinsichtlich ihrer konkreten Ausgestaltung, beispielsweise ihrer Größe, variiert werden, um verschiedene Implantatgrößen und Winkelungen zu schaffen, die eine größtmögliche Annährung an die individuelle Anatomie ermöglichen.

Die artikulierenden Gleitflächen 14, 15 sind vorzugsweise mit einer Co-Cr-Legierung beschichtet, was bei den in der Halswirbelsäule auftretenden Belastungen einen geringen Abrieb und somit ein langlebiges Bewegungsverhalten gewährleistet.

Die beiden Grundbauteile 1, 2, einschließlich der einteilig hiermit gebildeten Kopplungsbauteile 11, 12, sind bevorzugt aus Polyacryletherketon (PAEK) gefertigt, beispielsweise Polyetheretherketon (PEEK), Polyetherketon (PEK), Polyetherketonketon (PEKK), Polyetherketonetherketonketon (PEKEKK) oder Polyetherketonetherketon (PEKEK). Ein Vorteil von PAEK liegt in dessen Werkstoffeigenschaften, d.h. des ähnlichen Elastizitätsmoduls gegenüber kortikalen Knochen.

Zur weiteren Verbesserung der tribologischen und mechanischen Eigenschaften kann vorgesehen sein, PAEK mit einem Füllmaterial zu verwenden, beispielsweise Kohle- oder Glasfasern, und/oder die Polymermatrix zu modifizieren, zum Beispiel mittels Vernetzung oder einer Eisenimplantation.

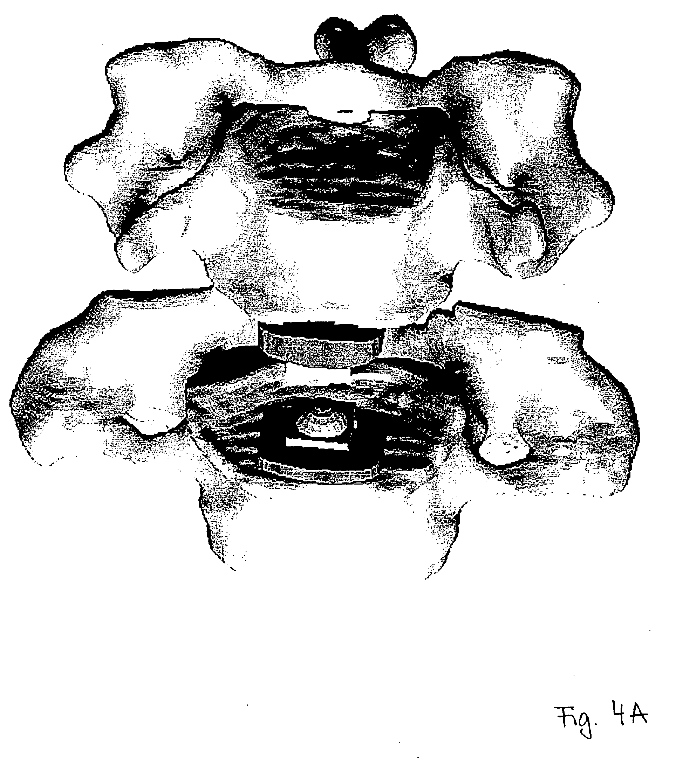

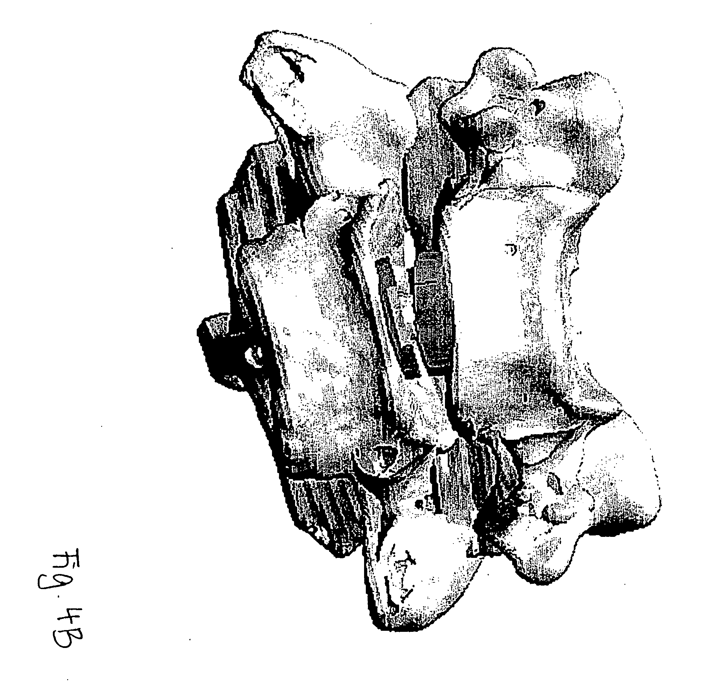

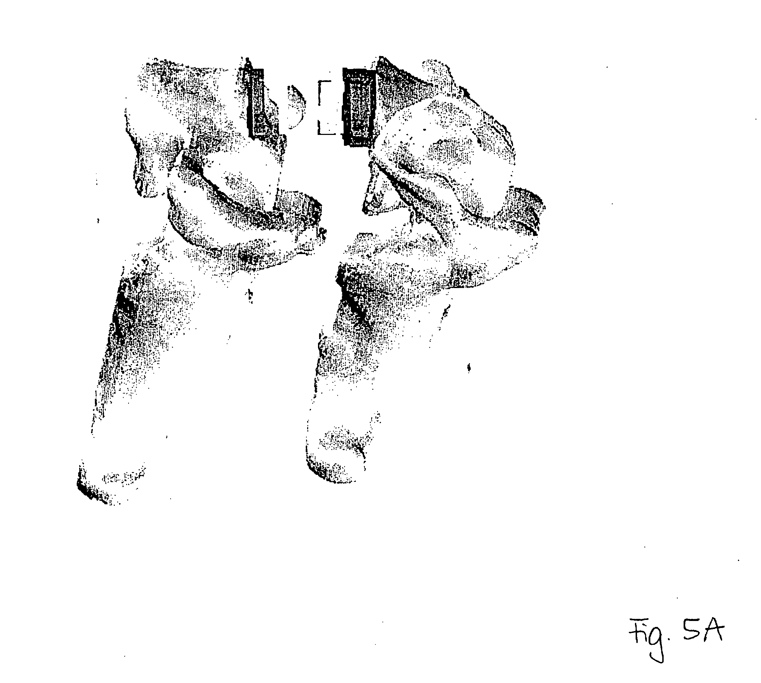

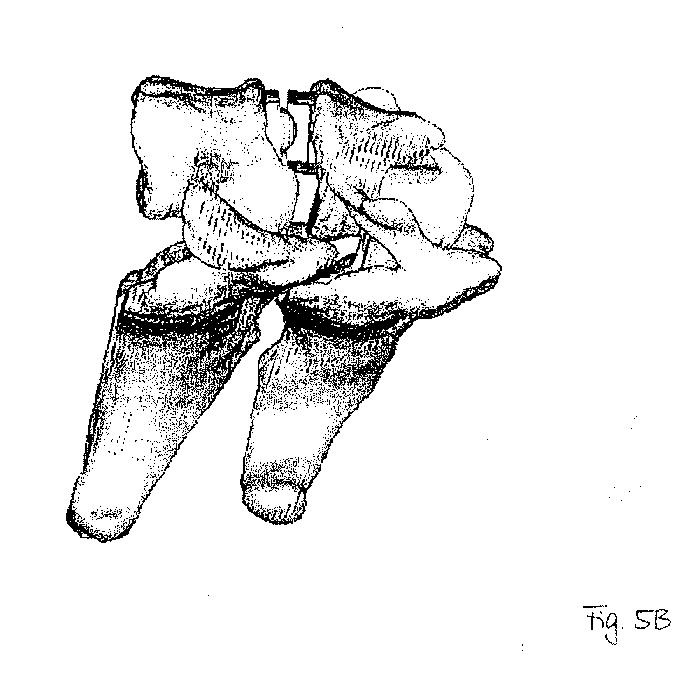

Die Fig. 4A, 4B und 5A, 5B zeigen perspektivische Darstellungen von Knochenteilen, die über eine Bauteilanordnung 30 gemäß Fig. 3 gelenkig miteinander verbunden sind, in einem entkoppelten Zustand und einem gekoppelten Zustand, wobei die Kopplungsteile bei der Darstellung in Fig. 4A aus den Grundbauteilen herausgelöst dargestellt sind.

Die beschriebenen Bauteile führen in Verbindung mit einer hierauf basierenden Halswirbelsäulen-Bandscheibenprothese zu den nachfolgend genannten Vorteilen gegenüber bekannten Prothesen: Rekonstruktion der Segmentbeweglichkeit; Rekonstruktion der individuellen Halswirbelsäulen-Lordose durch mögliche unterschiedliche Winkelung der Implantate; Rekonstruktion der individuellen Bandscheibenhöhe durch mögliche unterschiedliche Implantathöhen (modulare Systemtechnik); operativ-technische Vereinfachung des Vorgehens zur Einbringung des Implantates; Reduktion der Implantatkosten durch Zwei-Material-Technik und Modular-Technik; ermöglichte Anpassung der Prothesenform an die vorbestehenden anatomischen Verhältnisse; Vermeidung einer frühen Spontanfusion und dislokationssichere Implantation.

Das Implantationsinstrumentarium bei Verwendung einer Prothese auf Basis der beschriebnen Bauteile kann einfach gehalten werden und sieht neben Anpassungsdistraktoren und einem Implantathalter beispielsweise nur eine Deckplattenkürette und spezielle Halswirbelsäulen-Stanzen vor, die in der Lage sind, auch bei geringer Distraktion des Bewegungssegmentes eine knöcherne Dekompression auf der Dorsalseite des Wirbelkanals vorzunehmen.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Halswirbelsäulen-Bandscheibenprothese, mit zwei Grundbauteilen (1, 2), die mittels an den Grundbauteilen (1, 2) gebildeten Kopplungsbauteilen (11, 12) gelenkig miteinander gekoppelt sind, wobei die Grundbauteile (1, 2) jeweils mit einem zugehörigen Kopplungsbauteil (11, 12) einteilig gebildet sind, wobei die Grundbauteile (1, 2) und die Kopplungsbauteile (11, 12) aus einem Material sind, **dadurch gekennzeichnet, daß** das Material aus der folgenden Gruppe von Materialien ausgewählt ist: Polyetherketon (PEK), Polyacryletherketon (PAEK), Polyetheretherketon (PEEK), Polyetherketonketon (PEKK), Polyetherketonetherketonketon (PEKEKK) und Polyetherketonetherketon (PEKEK).

2. Halswirbelsäulen-Bandscheibenprothese nach Anspruch 1, **dadurch gekennzeichnet, daß** auf einer jeweiligen Außenseite (3, 4) der zwei Grundbauteile (1, 2) eine anatomisch angepaßte Kontaktfläche (5, 6) gebildet ist.

3. Halswirbelsäulen-Bandscheibenprothese nach Anspruch 1 oder 2, **dadurch gekennzeichnet, daß** an den zwei Grundbauteilen (1, 2) jeweils eine Verdrehsicherung gebildet ist.

4. Halswirbelsäulen-Bandscheibenprothese nach Anspruch 3, **dadurch gekennzeichnet, daß** die Verdrehsicherung einen auf der jeweiligen Außenseite (3, 4) angeordneten Steg (7, 8) umfaßt.

5. Halswirbelsäulen-Bandscheibenprothese nach einem der vorangehenden Ansprüche, **dadurch gekennzeichnet, daß** die zwei Grundbauteile (1, 2) mit Hilfe einer Gleitverbindung gelenkig miteinander gekoppelt sind.

6. Halswirbelsäulen-Bandscheibenprothese nach Anspruch 5, **dadurch gekennzeichnet, daß** die Gleitverbindung mit Hilfe einer an einem der Kopplungsbauteile (12) gebildeten Gleitfläche (14) und einer an die Gleitfläche (14) angepaßten Gegengleitfläche (15) an einem anderen der Kopplungsbauteile (11) ausgeführt ist, wobei die Gleitfläche im gekoppelten Zustand der zwei Grundbauteile (1, 2) auf der Gegengleitfläche gleitbar gelagert ist.

7. Halswirbelsäulen-Bandscheibenprothese nach Anspruch 6, **dadurch gekennzeichnet, daß** die Gleitfläche (14) auf einem halbkugelförmigen Vorsprung (13) an dem Kopplungsbauteil (12) gebildet ist.

8. Halswirbelsäulen-Bandscheibenprothese nach Anspruch 6 oder 7, **dadurch gekennzeichnet, daß** die Gleitfläche (14) und die Gegengleitfläche (15) mit einem Beschichtungsmaterial auf Basis einer Chrom-Nickel-Legierung beschichtet sind.

9. Halswirbelsäulen-Bandscheibenprothese nach einem der vorangehenden Ansprüche, **dadurch gekennzeichnet, daß** die zwei Grundbauteile (1, 2) und/oder die Kopplungsbauteile (11, 12) zumindest teilweise beschichtet sind.

10. Halswirbelsäulen-Bandscheibenprothese nach einem der Ansprüche 2 bis 9, **dadurch gekennzeichnet, daß** die anatomisch angepaßten Kontaktflächen (5, 6) und/oder die Stege (7, 8) eine Materialbeschichtung aufweisen.

11. Bauteil für eine Halswirbelsäulen-Bandscheibenprothese nach einem der vorangehenden Ansprüche, mit einem Grundbauteil (1; 2) und einem an dem Grundbauteil (1; 2) gebildeten Kopplungsbauteil (11; 12) für eine gelenkige Ankopplung eines anderen Grundbauteils (2; 1), wobei das Grundbauteil (1; 2) und das Kopplungsbauteil (11; 12) einteilig gebildet sind, **dadurch gekennzeichnet, daß** das Material aus der folgenden Gruppe von Materialien ausgewählt ist: Polyetherketon (PEK), Polyacryletherketon (PAEK), Polyetheretherketon (PEEK), Polyetherketonketon (PEKK), Polyetherketonetherketonketon (PEKEKK) und Polyetherketonetherketon (PEKEK).

12. Bauteil nach Anspruch 11, **gekennzeichnet durch** eine anatomisch angepaßte Kontaktfläche (5; 6) auf einer Außenseite (3; 4) des Grundbauteils (1; 2).

13. Bauteil nach Anspruch 11 oder 12, **gekennzeichnet durch** eine Verdrehsicherung auf der Außenseite (3; 4) des Grundbauteils (1,2).

14. Bauteil nach Anspruch 13, **dadurch gekennzeichnet, daß** die Verdrehsicherung einen auf der Außenseite (3; 4) angeordneten Steg (7; 8) umfaßt.

15. Bauteil nach einem der Ansprüche 11 bis 14, **dadurch gekennzeichnet, daß** an dem Kopplungsbauteil (11; 12) eine Gleitfläche (14; 15) gebildet ist.

16. Bauteil nach Anspruch 15, **dadurch gekennzeichnet, daß** die Gleitfläche (14; 15) gekrümmt ist.

17. Bauteil nach Anspruch 15 oder 16, **dadurch gekennzeichnet, daß** die Gleitfläche (14; 15) mit einem Material auf Basis einer Chrom-Nickel-Legierung beschichtet ist.

18. Bauteil nach einem der Ansprüche 11 bis 17, **gekennzeichnet durch** eine wenigstens teilweise Materialbeschichtung des Grundbauteils (1; 2) und/oder des Kopplungsbauteils (11; 12).

19. Bauteil nach einem der Ansprüche 12 bis 18, **dadurch gekennzeichnet, daß** die anatomisch angepaßten Kontaktflächen (5, 6) und/oder die Stege (7, 8) eine Materialbeschichtung aufweisen.

## Claims

1. A cervical spine intervertebral disc prosthesis, comprising two base parts (1, 2), which are coupled to one another in an articulated manner by means of coupling parts (11, 12) formed on the base parts (1, 2), wherein the base parts (1, 2) are in each case formed in one piece with an associated coupling part (11, 12), wherein the base parts (1, 2) and the coupling parts (11, 12) are made of one material **characterized in that** the material selected from the following group of materials: polyetherketone (PEK), polyetheretherketone (PEEK), polyacryletherketone (PAEK), polyetherketoneketone (PEKK), polyetherketoneetherketoneketone (PEKEKK) and polyetherketoneetherketone (PEKEK).

2. Cervical spine intervertebral disc prosthesis according to claim 1, wherein an anatomically adapted contact surface (5, 6) is formed on a respective outer side (3, 4) of the two base parts (1, 2).

3. Cervical spine intervertebral disc prosthesis according claim 1 or 2, wherein an anti-rotation means is formed on each of the two base parts (1, 2).

4. Cervical spine intervertebral disc prosthesis according to claim 3, wherein the anti-rotation means comprises a web (7, 8) arranged on the respective outer side (3, 4).

5. Cervical spine intervertebral disc prosthesis according to any one of the preceding claims, wherein the two base parts (1, 2) are coupled to one another in an articulated manner by means of a sliding connection.

6. Cervical spine intervertebral disc prosthesis according to claim 5, wherein the sliding connection is embodied by means of a sliding surface (14) formed on one of the coupling parts (12) and a countersliding surface (15), which is adapted to the sliding surface (14) and is formed on another of the coupling parts (11), wherein the sliding surface is slidably supported on the countersliding surface in the coupled state of the two base parts (1, 2).

7. Cervical spine intervertebral disc prosthesis according to claim 6, wherein the sliding surface (14) is formed on a hemispherical protrusion (13) on the coupling part (12).

8. Cervical spine intervertebral disc prosthesis according to claim 6 or 7, wherein the sliding surface (14) and the countersliding surface (15) are coated with a coating material based on a chromium-nickel alloy.

9. Cervical spine intervertebral disc prosthesis according to any one of the preceding claims, wherein the two base parts (1, 2) and/or the coupling parts (11, 12) are at least partially coated.

10. Cervical spine intervertebral disc prosthesis according to any of claims 2 to 9, wherein the anatomically adapted contact surfaces (5, 6) and/or the webs (7, 8) have a material coating.

11. A part for a cervical spine intervertebral disc prosthesis according to one of the preceding claims, comprising a base part (1; 2) and a coupling part (11; 12) formed on the base part (1; 2) for articulated coupling to another base part (2; 1), wherein the base part (1; 2) and the coupling part (11; 12) are formed in one piece, **characterized in that** the material is selected from the following group of materials: polyetherketone (PEK), polyetheretherketone (PEEK), polyacryletherketone (PAEK), polyetherketoneketone (PEKK), polyetherketoneetherketoneketone (PEKEKK) and polyetherketoneetherketone (PEKEK).

12. The part according to claim 11, wherein an anatomically adapted contact surface (5; 6) on an outer side (3; 4) of the base part (1; 2) is provided.

13. The part according to claim 11 or 12, wherein an anti-rotation means on the outer side (3; 4) of the base part (1; 2) is provided.

14. The part according to claim 13, wherein the anti-rotation means comprises a web (7; 8) arranged on the outer side (3; 4).

15. The part according to any of claims 11 to 14, wherein a sliding surface (14; 15) is formed on the coupling part (11; 12).

16. The part according to claim 15, wherein the sliding surface (14; 15) is curved.

17. The part according to claim 15 or 16, wherein the sliding surface (14; 15) is coated with a material based on a chromium-nickel alloy.

18. The part according to any of claims 11 to 17, wherein an at least partial material coating of the base part (1; 2) and/or of the coupling part (11; 12) is provided.

19. The part according to any of claims 12 to 18, wherein the anatomically adapted contact surfaces (5, 6) and/or the webs (7, 8) have a material coating.

## Revendications

1. Prothèse de disque intervertébral du rachis cervical comprenant deux éléments de base (1, 2) qui sont couplés l'un à l'autre de manière articulée au moyen d'éléments de couplage (11, 12) formés sur les éléments de base (1, 2), sachant que les éléments de base (1, 2) sont formés de manière monobloc avec chacun un élément de couplage (11, 12) correspondant, sachant que les éléments de base (1, 2) et les éléments de couplage (11, 12) sont fabriqués dans un matériau, **caractérisée en ce que** le matériau est choisi parmi le groupe de matériaux suivant : polyéthercétone (PEK), polyacryléthercétone (PAEK), polyétheréthercétone (PEEK), polyéthercétonecétone (PEKK), polyéthercétonétherecétonecétone (PEKEKK) et polyéthercétonétherecétone (PEKEK).

2. Prothèse de disque intervertébral du rachis cervical selon la revendication 1, **caractérisée en ce qu'**une surface de contact (5, 6) anatomiquement adaptée est formée sur un côté extérieur (3, 4) respectif des deux éléments de base (1, 2).

3. Prothèse de disque intervertébral du rachis cervical selon la revendication 1 ou 2, **caractérisée en ce qu'**une sécurité anti-rotation est formée respectivement sur les deux éléments de base (1, 2).

4. Prothèse de disque intervertébral du rachis cervical selon la revendication 3, **caractérisée en ce que** la sécurité anti-rotation comprend une branche (7, 8) placée sur le côté extérieur (3, 4) respectif.

5. Prothèse de disque intervertébral du rachis cervical selon l'une des revendications précédentes, **caractérisée en ce que** les deux éléments de base (1, 2) sont couplés l'un à l'autre de manière articulée au moyen d'une liaison coulissante.

6. Prothèse de disque intervertébral du rachis cervical selon la revendication 5, **caractérisée en ce que** la liaison coulissante est effectuée à l'aide d'une surface coulissante (14) formée sur un des éléments de couplage (12) et une contre-surface coulissante (15) adaptée à la surface coulissante (14) sur un autre des éléments de couplage (11), sachant que la surface coulissante est placée de manière coulissable sur la contre-surface coulissante lorsque les deux éléments de base (1, 2) sont couplés.

7. Prothèse de disque intervertébral du rachis cervical selon la revendication 6, **caractérisée en ce que** la surface coulissante (14) est formée sur une saillie (13) semi-sphérique sur l'élément de couplage (12).

8. Prothèse de disque intervertébral du rachis cervical selon la revendication 6 ou 7, **caractérisée en ce que** la surface coulissante (14) et la contre-surface coulissante (15) sont revêtues d'un matériau de revêtement à base d'un alliage de chrome-nickel.

9. Prothèse de disque intervertébral du rachis cervical selon l'une des revendications précédentes, **caractérisée en ce que** les deux éléments de base (1, 2) et/ou les éléments de couplage (11, 12) sont au moins partiellement revêtus.

10. Prothèse de disque intervertébral du rachis cervical selon l'une des revendications 2 à 9, **caractérisée en ce que** les surfaces de contact (5, 6) anatomiquement adaptées et/ou les branches (7, 8) présentent un revêtement de matériau.

11. Elément d'une prothèse de disque intervertébral du rachis cervical selon l'une des revendications précédentes, comprenant un élément de base (1 ; 2) et un élément de couplage (11 ; 12) formé sur l'élément de base (1 ; 2) pour un couplage articulé d'un autre élément de base (2 ; 1), sachant que l'élément de base (1 ; 2) et l'élément de couplage (11 ; 12) sont formés de manière monobloc, **caractérisé en ce que** le matériau est choisi parmi le groupe de matériaux : polyéthercétone (PEK), polyacryléthercétone (PAEK), polyétheréthercétone (PEEK), polyéthercétonecétone (PEKK), polyéthercétonétherecétonecétone (PEKEKK) et polyéthercétonétherecétone (PEKEK).

12. Elément selon la revendication 11, **caractérisé par** une surface de contact (5 ; 6) anatomiquement adaptée sur un côté extérieur (3 ; 4) de l'élément de base (1 ; 2).

13. Elément selon la revendication 11 ou 12, **caractérisé par** une sécurité anti-rotation sur le côté extérieur (3 ; 4) de l'élément de base (1 ; 2).

14. Elément selon la revendication 13, **caractérisé en ce que** la sécurité anti-rotation comprend une branche (7 ; 8) placée sur le côté extérieur (3 ; 4).

15. Elément selon l'une des revendications 11 à 14, **caractérisé en ce qu'**une surface coulissante (14 ; 15) est formée sur l'élément de couplage (11 ; 12).

16. Elément selon la revendication 15, **caractérisé en ce que** la surface coulissante (14 ; 15) est incurvée.

17. Elément selon la revendication 15 ou 16, **caractérisé en ce que** la surface coulissante (14 ; 15) est revêtue d'un matériau à base d'un alliage de chrome-nickel.

18. Elément selon l'une des revendications 11 à 17, **caractérisé par** un revêtement de matériau au moins partiel de l'élément de base (1 ; 2) et/ou de l'élément de couplage (11 ; 12).

19. Elément selon l'une des revendications 12 à 18, **caractérisé en ce que** les surfaces de contact (5, 6) anatomiquement adaptées et/ou les branches (7, 8) présentent un revêtement de matériau.
